# EUROPÄISCHE PATENTSCHRIFT

(11) **EP 1 291 697 B1**
(45) Veröffentlichungstag und Bekanntmachung des Hinweises auf die Patenterteilung: **16.11.2005**
(21) Anmeldenummer: 02019676.2
(22) Anmeldetag: 04.09.2002
(51) Int. Cl.: G02B 21/18, A61B 18/20, F16C 11/10

(54) **Mikroskoptubus mit mindestens zwei Drehgelenken und mechanischer Blockiereinrichtung**
Microscope tube with at least two rotary joints and mechanical blocking system
Tube de microscope avec au moins deux articulations rotatives et un système de blocage mécanique

(30) Priorität: 07.09.2001 DE 10144033
(43) Veröffentlichungstag der Anmeldung: 12.03.2003
(73) Patentinhaber: Carl Zeiss Surgical GmbH, 73447 Oberkochen (DE)
(72) Erfinder: Weber, Lauric, 73431 Aalen (DE); Koch, Werner, 75210 Keltern (DE); Robra, Wolfgang, 75323 Bad Wildbad (DE)

(56) Entgegenhaltungen:
- EP-A- 1 120 676
- DE-A- 2 713 737
- US-A- 3 638 973
- US-A- 4 270 845
- US-A- 5 564 667
- PATENT ABSTRACTS OF JAPAN vol. 2000, no. 08, 6. Oktober 2000 (2000-10-06) -& JP 2000 139949 A (OLYMPUS OPTICAL CO LTD), 23. Mai 2000 (2000-05-23) -& US 2002/151784 A1 17. Oktober 2002 (2002-10-17)

## Beschreibung

Die vorliegende Erfindung betrifft einen Beobachtertubus für ein Mikroskop mit mindestens zwei Drehgelenken.

Ein Beobachtertubus der eingangs genannten Art ist aus der EP 120 676 A2 bekannt. Dort ist ein Stereomikroskop beschrieben, das einen Beobachtungstubus aufweist, der um eine erste Achse gedreht und um eine von der ersten Achse verschiedenen zweiten Achse geschwenkt werden kann.

Die US 3,638,973 offenbart einen Mehrgelenkarm zur Manipulation eines Wundhakens durch einen Chirurgen. In dem Mehrgelenkarm ist ein Leitungssystem für Fluid angeordnet. Mittels eines Betätigungselements kann ein Druck des Fluids in dem Leitungssystem vergrößert oder verkleinert werden. Wird der Druck des Fluids in dem Leitungssystem vergrößert, so werden an den Gelenken des Mehrgelenkarmes zueinander bewegliche Teile in Wirkeingriff gebracht, um so eine Bewegung des entsprechenden Gelenkes zu unterbinden. Umgekehrt wird eine Gelenkbewegung bei Verringerung des Druckes im Leitungssystem freigegeben.

In der DE 27 13 737 ist ein Gelenklichtleiter beschrieben, mit dem Laserlicht durch röhrenförmig Verbindungsstücke geführt werden kann. Zur Umlenkung von Laserlicht in den Gelenken des Gelenklichtleiters sind Spiegel vorgesehen. Um eine Drehbewegung der Gelenke des Gelenklichtleiters zu dämpfen, sind Drehlagern beim Gelenklichtleiter Bremseinrichtungen zugeordnet.

Die JP 2001139949 A offenbart ein Operationsmikroskop, das an einem Stativ mit mehreren Drehgelenken aufgenommen ist. An den Drehgelenken des Stativs sind elektromagnetische Bremsen vorgesehen, welche geöffnet und geschlossen werden können.

In der US 5,564,667 ist ein mehrgelenkiger Tragarm beschrieben, der eine Plattform hält. Der Tragarm umfasst mehrere Drehgelenke. Den Drehgelenken im Tragarm sind Bremseinrichtungen zugeordnet. Diese Bremseinrichtungen können mittels eines Seilzuges gleichzeitig gelöst bzw. gesperrt werden.

Die DE 33 33 471 C2 beschreibt ein Operationsmikroskop für zwei Operateure bekannt, das einen Beobachtungstubus für den Operateur und einen zweiten Beobachtungstubus für den Assistenten aufweist. Der Beobachtungstubus für den Assistenten weist eine senkrecht zur Tubusachse liegende Drehgelenkstelle auf, um die der Okulareinblick um 360° relativ zum Basisteil des Mitbeobachtertubus, mit dem der Mitbeobachtertubus am Operationsmikroskop aufgenommen ist, drehbar ist. Darüberhinaus weist der Mitbeobachtertubus eine weitere Drehgelenkstelle auf, um die der Mitbeobachtertubus um die optische Achse des Hauptbeobachtertubus drehbar ist.

Die Drehgelenke des Mitbeobachtertubus dienen dazu, daß der Assistent seinen Okulareinblick relativ zum Hauptkörper des Operationsmikroskopes in eine für ihn ergonomisch günstige Position bringen kann. Hinsichtlich der Reibkräfte in den Drehgelenken bestehen einander widersprechende Forderungen: Einerseits sollen die Reibkräfte so hoch sein, daß die Drehgelenke in einer eingestellten Position verharren und auch nicht durch geringfügige Kräfte unbeabsichtigt in eine andere Position verschwenkt werden; andererseits soll in Fällen, in denen eine geänderte Verdrehung der Tubusteile zueinander gewünscht ist, die Verdrehung mit möglichst geringen Kräften möglich sein und nach Möglichkeit eine Einhandbedienung ermöglichen.

Diese einander widersprechenden Forderungen werden erfindungsgemäß durch einen *Beobachtertubus* mit den Merkmalen des Anspruches 1 gelöst. Vorteilhafte Ausgestaltungen der Erfindung ergeben sich aus den Merkmalen der abhängigen Ansprüche.

Der erfindungsgemäße Tubus weist mindestens zwei Drehgelenke auf. Die Drehgelenke sind mittels Bremsen arretierbar. Für die Lösung der Bremsen ist ein gemeinsamer Bedienhebel vorgesehen, bei dessen Betätigung beide Drehgelenke - bei mehr als zwei Drehgelenken alle Drehgelenke - freigegeben sind.

Bei nicht betätigtem Bedienhebel sind die Drehgelenke mittels der Bremsen arretiert. Eine unbeabsichtigte Verdrehung der Drehgelenke ist dadurch ausgeschlossen. Ist hingegen eine Verdrehung gewünscht, so kann der Benutzer die Bremsen lösen; da bei gelösten Bremsen die Drehgelenke nicht mehr gehemmt sind, kann die Verdrehung mit geringstem Kraftaufwand erfolgen. Dadurch daß bei einer Betätigung des Bedienhebels alle Bremsen gleichzeitig gelöst werden, ergibt sich der Bedienungsvorteil, daß sich der Bediener nicht im voraus überlegen muß, um welche Drehgelenkstelle eine Verdrehung erfolgen muß, um den Okulareinblick in die gewünschte Position zu bringen. Der Bediener muß vielmehr lediglich den einzigen Bedienungshebel betätigen und nachfolgend den Okulareinblick in die gewünschte neue Position verschwenken und in dieser neuen Position die Bremsen nachfolgend - durch Loslassen des Bedienhebels - wieder arretieren.

Der Bedienhebel für die Bremsen ist vorzugsweise okularseitig oder benutzerseitig am Tubus angeordnet.

Die Übertragung der Bewegung des Bedienhebels erfolgt rein mechanisch über Stössel und Umlenkhebel wobei dann auch die Bremsen selbst rein mechanisch ausgebildet sein können.

Bei einer bevorzugten Ausführungsform einer mechanischen Übertragung der Bewegung des Bedienhebels auf die Bremsen sind die Bremsen durch eine Kraftbeaufschlagung parallel zur jeweiligen Drehachse ohne weitere Kraftumlenkung lösbar. Dadurch ergibt sich eine konstruktiv einfache Lösung. Bei einer Ausführungsform, die keine weitere Kraftumlenkung erfordert, bestehen die Bremsen aus Lamellen mit Reibbelägen und Mitnehmern, wobei die Mitnehmer aufeinander folgender Lamellen alternierend in das Innenrohr und das Außenrohr der gegeneinander drehbaren Teile des Drehgelenkes eingreifen. Dabei ist es möglich, die verschiedenen Drehgelenke auch mit unterschiedlicher Bremskraft zu arretieren, indem die Bremsen der verschiedenen Drehgelenke unterschiedliche Anzahlen an Lamellen aufweisen.

Zur Erzeugung der Bremskraft sind die Bremsen bzw. die Stössel vorzugsweise mittels ein oder mehrerer Druckfedern mit dem Bremsdruck beaufschlagt.

Bei einer alternativen Ausgestaltung der Bremsen sind zwischen dem Innenrohr und dem Außenrohr eines jeden Drehgelenkes Rollen vorgesehen, deren Achsen sich parallel zur Tubusachse erstrecken und die durch Federn entlang einer v-förmigen Fläche am Außenröhr gegen das Innenrohr gedrückt werden. Zum Lösen der Bremsen weisen dann die Stössel im Bereich der Rollen angeschrägte Flächen auf, durch die die Rollen bei Betätigung des Bedienhebels gegen die Kraft der Druckfedern nach außen gedrückt werden. Derartige Klemmrollen-Freilaufbremsen ermöglichen extrem hohe Haltekräfte und sind spielfrei, fuhren aber auch zu hohen radialen Kräften auf die Lagerstellen der Drehgelenke. Außerdem sind die Haltekräfte nicht dosierbar, eine Verdrehung der Drehgelenke bei arretierter Bremse ist nahezu ausgeschlossen und es ist nicht ohne weiteres möglich, unterschiedliche Bremskräfte für die verschiedenen Drehgelenke vorzusehen.

Nachfolgend werden Einzelheiten der Erfindung anhand des in den Figuren dargestellten Ausführungsbeispiels näher erläutert. Dabei zeigen:
- Figur 1: ein Operationsmikroskop mit einem erfindungsgemäßen Tubus im Schnitt, wobei die Schnittebene die optische Achse enthält,
- Figur 2: einen Schnitt durch den Mitbeobachtertubus in Figur 1 in einer zur Figur 1 parallelen Schnittebene,
- Figur 3: den Schnitt senkrecht zur Tubusachse einer alternativen Ausführungsform der Erfindung; und
- Figur 4: den Schnitt durch die Ausführungsform in Figur 3 in einer zur Figur 3 senkrechten Schnittebene.

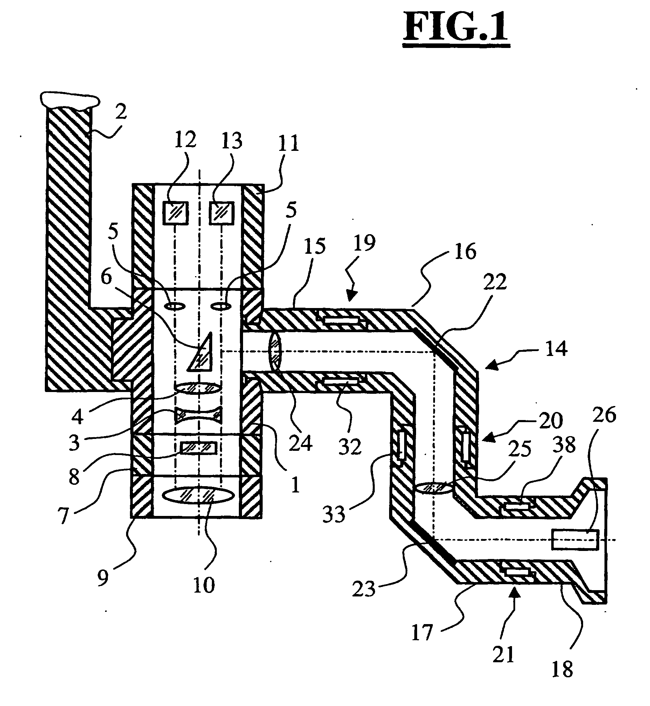

Das Operationsmikroskop in der Figur 1 weist einen Mikroskop-Grundkörper (1) auf, mit dem es an einem hier nicht näher dargestellten Stativ (2) aufgenommen ist. Der Mikroskop-Grundkörper enthält einen Vergrößerungswechsler (3, 4) oder ein Zoomsystem zur Änderung der Vergrößerung. Weiterhin sind im Mikroskop-Grundkörper oder einem daran aufgenommenen separaten Modul zwei Umlenkprismen (6) vorgesehen, mit deren Hilfe der Beobachtungsstrahlengang in Richtung auf den Mitbeobachtertubus (14) umgelenkt wird.

Unterhalb des Mikroskop-Grundkörpers schließt sich an diesen ein Beleuchtungsmodul (7) mit einer nicht näher dargestellten Einkoppeloptik für die Beleuchtung und Einkoppelspiegeln (8) an. Auf das Beleuchtungsmodul (7) folgt das Objektivmodul (9) mit dem darin aufgenommenen Hauptobjektiv (10). Oberhalb des Mikroskop-Grundkörpers (1) ist der Okulartubus (11) für den Hauptbeobachter mit den beiden Tubuslinsen (5) für die beiden stereoskopischen Teilstrahlengänge (die teilweise in den Mikroskopörper hineinragen können) und separaten Umlenkprismen (12, 13) angeordnet. Nicht mehr dargestellt in der Figur 1 ist der eigentliche Mikroskopeinblick für den Hauptbeobachter, der in einer zur Zeichenebene in Figur 1 parallelen Ebene liegen würde.

Seitlich am Mikroskophauptkörper (1) oder an einem daran aufgenommenen Teilermodul ist der Mitbeobachtertubus (14) angeschlossen. Dieser hat eine im wesentlichen z-förmige Gestalt und besteht aus vier Rohrteilen (15, 16, 17, 18), von denen die beiden mittleren Rohrteile (16, 17) Bogenstücke sind. Das erste Rohrteil (15) ist mit einem Ende am Mikroskopgrundkörper (1) aufgenommen. Das als erstes Bogenstück ausgebildete zweite Rohrteil (16) ist mit einem Ende am zweiten Ende des ersten Rohrteils (15) um die Rohrachse des Rohrteiles (15) drehbar aufgenommen, so daß zwischen dem zweiten Rohrteil (16) und dem ersten Rohrteil (15) ein erstes Drehgelenk (19) besteht. Das als zweites Bogenstück (17) ausgebildete dritte Rohrteil ist mit einem Ende am zweiten Ende des zweiten Rohrteils (16) um die abgewinkelt zur Rohrachse des ersten Rohrteiles (15) verlaufende Rohrachse des zweiten Rohrteiles (16) drehbar aufgenommen, so daß zwischen dem zweiten Bogenteil und dem ersten Bogenteil ein zweites Drehgelenk (20) besteht. Am anderen Ende des dritten Rohrteiles (17) schließt sich der Okularstutzen (18) an, der wiederum um die Rohrachse des zweiten abgewinkelten Rohrteiles (17) drehbar ist, so daß zwischen dem vierten Rohrteil und dem dritten Rohrteil ein drittes Drehgelenk (21) besteht. Das erste und das dritte Drehgelenk (19, 21) liegen dabei in zueinander parallelen voneinander beabstandeten Ebenen, während das dazwischenliegende zweite Drehgelenk eine Drehung um eine zu den beiden anderen Drehgelenken senkrechte Ebene gestattet. Eine Drehung um das erste Drehgelenk (19) dient dabei zur Einstellung der Einblickhöhe in den Mitbeobachtertubus, die Drehung um das zweite Drehgelenk (20) zur Einstellung der Einblickrichtung in den Mitbeobachtertubus (14) und die Drehung um das dritte Drehgelenk (21) zur Einstellung des Okulareinblickes entsprechend der Kopfneigung des Mitbeobachters und/oder daß die optischen Achsen der beiden Okular (26) des Mitbeobachtertubus (14) in einer im wesentlichen horizontalen Ebene liegen. Um alle drei Drehgelenke (19, 20, 21) kann eine Drehbarkeit um 360° möglich sein.

Im Inneren freien Raum des Mitbeobachtertubus (14) sind Umlenkspiegel (22, 23) (jeweils in den Bogenstücken (16, 17)) und Linsensysteme (24, 25), die gemeinsam ein afokales System mit Zwischenbild bilden, und zwei Prismen (26) zur Aufteilung des Strahlenganges in die beiden Okulare des anzusetzenden (nicht mit dargestellten) Okulartubüs für den Mitbeobachter angeordnet.

Zur Vermeidung unbeabsichtigter Verdrehungen um die Drehgelenke (19, 20, 21) sind in den Drehgelenken Bremsen (32, 33, 38) vorgesehen. Im dargestellten Ausführungsbeispiel bestehen die Bremsen (32, 33, 38) aus schwimmend gelagerten, ringförmigen Lamellen, die mit Reibbelägen beschichtet sind. Die jeweils äußeren Lamellen sowie jede zweite darauf folgende Lamelle greift über Mitnehmer in das jeweilige Innenrohr des Drehgelenkes ein und jede zweite verbleibende Lamelle greift über Mitnehmer in das jeweilige Außenrohr des Drehgelenkes ein. Der Eingriff der Lamellen in das jeweilige Außenrohr oder Innenrohr des Drehgelenkes ist dabei in azimutaler Richtung - also bezüglich Drehungen um die jeweilige Rohrachse - nahezu spielfrei, während die Lamellen in Richtung der jeweiligen Rohrachse schwimmend gelagert sind und in dieser Richtung Spiel aufweisen. Bei arretierten Bremsen ist besteht dadurch nur ein sehr geringes Spiel in den Drehgelenken.

Die beiden äußeresten Lamellen eines jeden Lammellenpaketes, das jeweils eine Bremse darstellt, greifen entweder beide in das Außenrohr oder beide in das Innenrohr ein. Die Anzahl der Lamellen in einem jeden Lamellenpaket, das gemeinsam eine Bremse darstellt, ist demzufolge ungerade. Die Anzahl der Lamellen in jedem der drei Drehgelenke (19, 20, 21) kann dabei unterschiedlich gewählt sein, da über die Anzahl der Lamellen die Reibkraft der Bremse im arretierten Zustand definiert wird. Das erste Drehgelenk (19) weist dabei die größte Anzahl an Lamellen (32) auf, so daß bei arretierter Bremse die Drehung um dieses Drehgelenk (19) am stärksten gehemmt ist, da an diesem Drehgelenk die größten Momente auftreten können. Die Anzahl der Lamellen der in Richtung auf den Okulareinblick nachfolgenden Lamellenpaketen (33, 38) nimmt in Richtung auf den Okulareinblick (26) ab, d.h. die Bremswirkung im zweiten Drehgelenk (20) ist geringer als die Bremswirkung im ersten Drehgelenk (19) und die Bremswirkung im dritten Drehgelenk (21) geringer als die Bremswirkung im zweiten Drehgelenk (20).

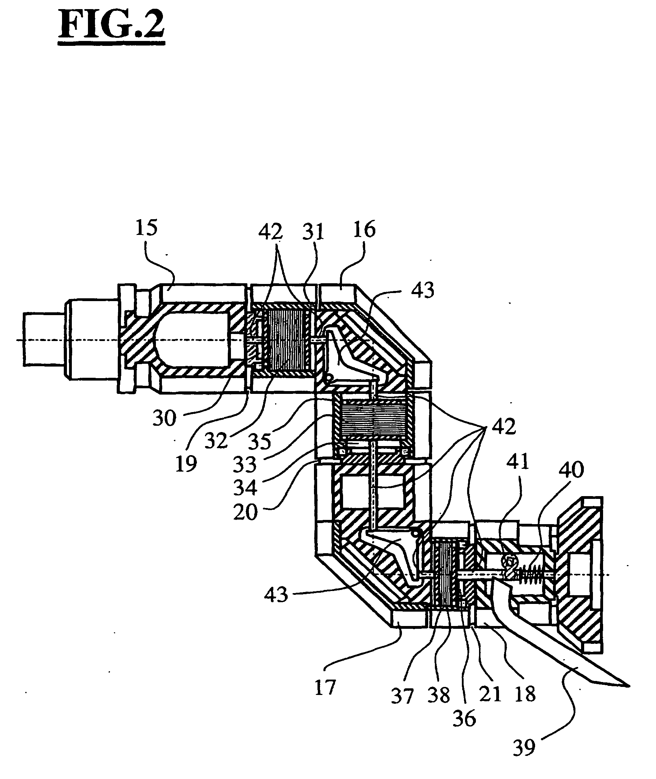

Alle drei Bremsen (32, 33, 38) sind durch Betätigung eines gemeinsamen Bedienhebels (39) (Figur 2) lösbar. Der Bedienhebel (39) ist dabei aus ergonomischen Gründen okularseitig des Tubus (14) am vierten Rohrteil (18) angeordnet. Die Mechanik sowohl zur Übertragung einer Betätigung des Bedienhebels (39) als auch zur Beaufschlagung der Bremsen mit dem erforderlichen Bremsdruck erfolgt über ein System von Stössel und Umlenkhebeln, die in der Rohrwand des Tubus (14) angeordnet sind und nachfolgend anhand der Figur 2 näher erläutert werden.

Wie bereits weiter oben gesagt, besteht jede der Bremsen (32, 33, 38) aus einem Paket von ringförmigen Lamellen, die abwechselnd über Mitnehmer in das Innenrohr (30, 34, 36) und in das Außenrohr (31, 35, 37) des jeweiligen Drehgelenkes eingreifen. Die Lamellenpakte sind auf beiden Seiten jeweils durch Ringgscheiben begrenzt, von denen jeweils eine schwimmend am Außenrohr und die andere schwimmend am Innenrohr des Drehgelenkes aufgenommen ist. Alle drei Bremsen sind über ein System aus Stösseln (42), deren Bewegung in den Bogenstücken (16, 17) mittels Kipphebel (43) umgelenkt wird, miteinander verbunden. Zur Beaufschlagung aller drei Bremsen (32, 33, 38) mit einem einheitlichen Bremsdruck ist das System aus Stösseln und Umlenkhebeln von einer Seite über eine Druckfeder (40) vorgespannt. Dabei drückt die Druckfeder (40) auf das okularseitige Ende des Systems aus Stösseln (42) und Kipphebeln (43). Der okularseitige Stössel drückt dabei auf die Bremse (38) des dritten Drehgelenkes und drückt die zugehörigen Lamellen der Bremse (38) zusammen. Über das nachfolgende System aus Stösseln (42) und Kipphebeln (43) wird der von der Druckfeder (40) erzeugte Bremsdruck auf die zweite Bremse (33) und von dort über zwei weitere Stössel und einen Kipphebel (43) auf die mikroskopseitige Bremse (32) übertragen. Ein weiteres Stösselstück, kann im Innenrohr des ersten Rohrabschnittes (15) aufgenommen sein und als Widerlager dienen. Bei einer Drehung um die Drehgelenke gleiten die Stößel jeweils über die Oberfläche der am Innenrohr des Drehgelenkes schwimmend aufgenommenen Ringscheibe, so daß die Übertragung des Bremsdruckes über das Stößel-Kipphebel-System in allen Drehstellungen gewährleistet ist.

Der Bedienhebel (39) greift über einen Mitnehmer (41) zwischen der Druckfeder (40) und der okularseitigsten dritten Bremse (38) in das System aus Stösseln (42) und Kipphebeln (43) ein. Bei Betätigung des Bedienelementes (39) wird das okularseitige Ende des Stössel-Kipphebel-Systems gegen die Kraft der Druckfeder (40) in Richtung auf das Okular bewegt. Dadurch wird der von der Druckfeder (40) erzeuge Bremsdruck zurückgenommen. Durch die serielle Anordnung der Bremsen (32, 33, 38) werden dadurch alle drei Bremsen gleichzeitig gelöst.

Damit die schwimmend gelagerten Lamellen der Bremsen (32, 33, 38) nicht verkanten, sind zwei Systeme aus Stösseln (42) und Kipphebeln (43) in zwei zueinander parallelen, bezogen auf die Rohrachse um 180° versetzt zu einander liegenden Ebenen vorgesehen.

Der Bedienhebel (39) ist an dem dem Benutzer zugewandten, okularseitigen letzten Rohrteil angeordnet und verbindet beide Stößel-Kipphebelsysteme. Die Prismen zur Aufteilung des Strahlenganges in die beiden Okulare sind in derselben Ebene angeordnet wie die Eingriffe des Bedienhebels in das Stößel-Kipphebelsystem im letzten Rohrteil. Dadurch bleibt der Bedienhebel auch bei einer Drehung um die Drehgelenke bezogen auf die Orientierung des Okulartubus gleich angeordnet.

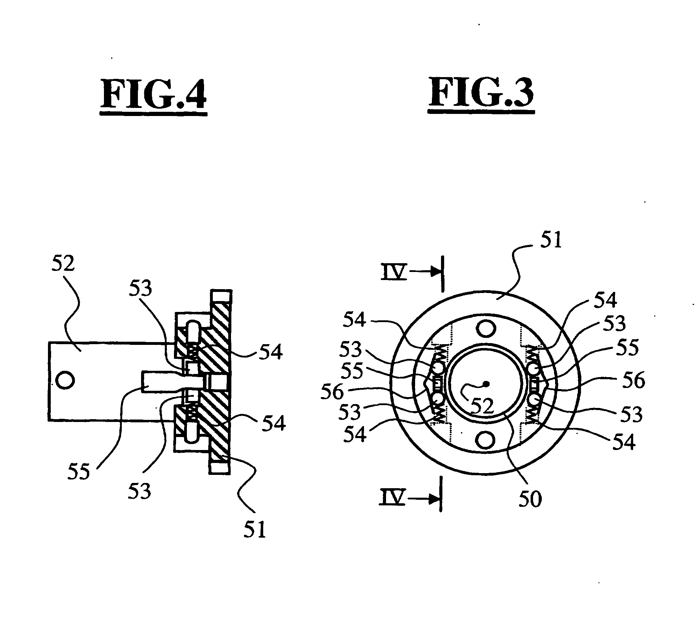

In den Figuren 3 und 4 ist eine alternative Ausführungsform für die Bremsen in den Drehgelenken dargestellt. Hierbei handelt es sich um sogenannte Klemmrollen-Freilaufbremsen. Für diese Art der Bremsen weist das Außenrohr (51) des Drehgelenkes annähernd v-förmige Innenflächen (55) auf, in die Rollen (53) mit ihren Achsen parallel zur Drehachse (52) des Drehgelenkes eingesetzt sind. Weiterhin weist die Bremse Federn (54) auf, durch die die Rollen (53) senkrecht zu den Rollenachsen mit einer Druckkraft beaufschlagt werden. Durch die Druckkraft der Federn (54) werden die Rollen (53) entlang der v-förmigen Flächen (56) des Innenrohres (51) gedrückt und klemmen so das Drehgelenk fest. Zwischen den Rollen (53) sind die Betätigungsstangen (55) angeordnet, deren Achsen sich ebenfalls parallel zur Drehachse (52) des Drehgelenkes erstrecken und im Bereich der Rollen (53) angeschrägte, den Rollen (53) zugewandte Flächen aufweisen. Bei Betätigung der Betätigungsstangen (55) drücken diese die Rollen (53) auseinander wodurch die Bremse des Drehgelenkes gelöst wird. Die zu mehreren seriell hintereinander geschalteten Drehgelenken zugehörigen Bremsen können auch bei dieser Ausführungsform seriell miteinander verkoppelt sein, indem die Betätigungsstangen (55) als ein System aus Stösseln und Kipphebeln, die die zu den hintereinander geschalteten Drehgelenken zugehörigen Bremsen miteinander verbinden, ausgebildet sind oder über ein entsprechendes System aus Stösseln und Kipphebeln betätigt werden. Im Gegensatz zu der anhand der Figuren 1 und 2 beschriebenen Ausführungsform der Bremsen durch ringförmige Lamellen ist bei einer solchen Klemmrollen-Freilaufbremse allerdings die Haltekraft der Bremse nicht dosierbar, d.h., alle Drehgelenke weisen die selbe Schwergängigkeit bei arretierter Bremse auf und auch eine beabsichtigte Verdrehung in den Drehgelenken bei angezogener Bremse ist nicht möglich oder führt eventuell zu Beschädigungen an den gegeneinander klemmenden Flächen.

Ein mit Klemmrollen-Freilaufbremsen ausgestattetes Ausführungsbeispiel der Erfindung hat abgesehen von der andersartigen Gestaltung der Bremsen selbst ansonsten einen zu der Ausführungsform in Figuren 1 und 2 völlig analogen Aufbau, so daß in soweit auf die obige Figurenbeschreibung verwiesen sei.

## Patentansprüche

1. Beobachtungstubus für ein Mikroskop
mit mindestens zwei Drehgelenken (19, 20, 21)
**dadurch gekennzeichnet, dass**
der Beobachtertubus mindestens zwei Bremsen (32, 33, 38) zum Feststellen der Drehgelenke aufweist; wobei
den Bremsen (32, 33, 38) ein gemeinsames Bedienelement (39) zugeordnet ist, das bewegt werden kann und mittels dessen die Bremsen (32, 33, 38) gleichzeitig gelöst werden können; und wobei
Stößel (42) und Kipphebel (43) zur Übertragung einer Bewegung des Bedienelements (39) auf die Bremsen (32, 33, 38) vorhanden sind.

2. Beobachtertubus nach Anspruch 1, **dadurch gekennzeichnet, dass** die Bremsen (32, 33, 38) mittels ein oder mehrerer Druckfedern (40) mit Bremsdruck beaufschlagt sind.

3. Beobachtertubus nach Anspruch 2, **dadurch gekennzeichnet, dass** die ein oder mehreren Druckfedern (40) auf das System von Stößeln (42) und Kipphebeln (43) wirken, wobei das Bedienelement (39) zwischen den ein oder mehreren Druckfedern (40) und der den ein oder mehreren Druckfedern (40) am nächsten benachbarten Bremse (38) in das System aus Stößeln (42) und Kipphebeln (43) eingreift.

4. Beobachtertubus nach einem der Ansprüche 1 bis 3, **dadurch gekennzeichnet, dass** jede der Bremsen (32, 33, 38) einem Drehgelenk (19, 20, 21) zugeordnet ist und durch eine Kraftbeaufschlagung parallel zu einer Drehachse des Drehgelenks ohne weitere Kraftumlenkung lösbar ist.

5. Beobachtertubus nach einem der Ansprüche 1 bis 4, **dadurch gekennzeichnet, dass** der Tubus an den Drehgelenken (19, 20, 21) jeweils ein Innenrohr (30, 34, 36) und ein Außenrohr (31, 35, 37) aufweist und die Bremsen aus Lamellen (32, 33, 38) mit Reibbelägen und Mitnehmern bestehen, die alternierend in das Innenrohr (30, 34, 36) und das Außenrohr (31, 35, 37) eingreifen.

6. Beobachtertubus nach Anspruch 5, **dadurch gekennzeichnet, dass** die Bremsen (32, 33, 38) der verschiedenen Drehgelenke (19, 20, 21) unterschiedliche Anzahlen an Lamellen aufweisen.

7. Beobachtertubus nach einem der Ansprüche 1 bis 6, **dadurch gekennzeichnet, dass** die Bremsen sich parallel zur Tubusachse (52) im Bereich des Drehgelenkes erstreckende Rollen (53) aufweisen, die durch Federn (54) gegen das Innenrohr (52) gedrückt werden.

8. Beobachtertubus nach einem der Ansprüche 1 bis 7, **dadurch gekennzeichnet, dass** drei Drehgelenke (19, 20, 21) vorgesehen sind, wobei die Drehachsen zweier Drehgelenke parallel zueinander, voneinander beabstandet sind und die Drehachsen eines dazwischenliegenden Drehgelenkes senkrecht zu den beiden anderen Drehachsen steht.

9. Beobachtertubus nach einem der Ansprüche 1 bis 8, **dadurch gekennzeichnet, dass** im Innenraum des Tubus optische Komponenten (22, 23) zur Übertragung eines optischen Strahlenganges vorgesehen sind.

10. Beobachtertubus nach einem der Ansprüche 1 bis 9, **dadurch gekennzeichnet, dass** das System aus Stößeln und Kipphebeln in der Wand des Tubus angeordnet ist.

11. Beobachtertubus nach einem der Ansprüche 1 bis 10, **dadurch gekennzeichnet, dass** der Beobachtertubus ein Okular (26) umfasst, der in einem Rohrteil (18) aufgenommen ist, an dem das Bedienelement (39) angeordnet ist.

12. Beobachtertubus nach einem der Ansprüche 1 bis 11, **dadurch gekennzeichnet, dass** ein Prismenpaar (26) zur Aufteilung eines Strahlenganges in Okulare eines angesetzten oder anzusetzenden Okulartubus vorgesehen ist.

## Claims

1. Microscope observing tube with at least two rotary joints (19, 20, 21), **characterized in that** the observing tube has at least two brakes (32, 33, 38) for fixing the rotary joints, in which the brakes (32, 33, 38) are assigned a common operating control (39) which can be moved and by means of which the brakes (32, 33, 38) can be released simultaneously, and in which plungers (42) and tilt levers (43) are present for transmitting a movement of the operating control (39) onto the brakes (32, 33, 38).

2. Observing tube according to Claim 1, **characterized in that** brake pressure is applied to the brakes (32, 33, 38) by means of one or more compression springs (40).

3. Observing tube according to Claim 2, **characterized in that** the one or more compression springs (40) act on the system of plungers (42) and tilt levers (43), the operating control (39) engaging in the system of plungers (42) and tilt levers (43) between the one or more compression springs (40) and the one or more compression springs (40) on the most closely adjacent brake (38).

4. Observing tube according to one of Claims 1 to 3, **characterized in that** each of the brakes (32, 33, 38) is assigned to a rotary joint (19, 20, 21) and can be released by applying force in a fashion parallel to an axis of rotation of the rotary joint without further force deflection.

5. Observing tube according to one of Claims 1 to 4, **characterized in that** the tube respectively has an inner conduit (30, 34, 36) and an outer conduit (31, 35, 37) at the rotary joints (19, 20, 21), and the brakes comprise discs (32, 33, 38) with friction linings and drivers which alternately engage in the inner conduit (30, 34, 36) and the outer conduit (31, 35, 37).

6. Observing tube according to Claim 5, **characterized in that** the brakes (32, 33, 38) of the various rotary joints (19, 20, 21) have different numbers of discs.

7. Observing tube according to one of Claims 1 to 6, **characterized in that** the brakes have rollers (53) which extend parallel to the tube axis (52) in the region of the rotary joint and are pressed against the inner conduit (52) by springs (54).

8. Observing tube according to one of Claims 1 to 7, **characterized in that** three rotary joints (19, 20, 21) are provided, the axes of rotation of two rotary joints being spaced apart in a parallel fashion from one another, and the axes of rotation of a rotary joint situated therebetween being perpendicular to the other two axes of rotation.

9. Observing tube according to one of Claims 1 to 8, **characterized in that** optical components (22, 23) are provided in the interior of the tube in order to transmit an optical beam path.

10. Observing tube according to one of Claims 1 to 9, **characterized in that** the system of plungers and tilt levers is arranged in the wall of the tube.

11. Observing tube according to one of Claims 1 to 10, **characterized in that** the observing tube includes an eyepiece (26), which is held in a tubular part (18) on which the operating control (39) is arranged.

12. Observing tube according to one of Claims 1 to 11, **characterized in that** a pair (26) of prisms are provided for splitting a beam path into eyepieces of an eyepiece tube which has been or is to be attached.

## Revendications

1. Tube d'observation pour un microscope comprenant au moins deux articulations tournantes (19, 20, 21) ; **caractérisé en ce que** le tube d'observation présente au moins deux freins (32, 33, 38) pour bloquer les articulations tournantes ; un élément de commande commun (39) étant associé aux freins (32, 33, 38), lequel peut être déplacé et au moyen duquel les freins (32, 33, 38) peuvent être desserrés simultanément ; et un coulisseau (42) et un levier coudé (43) étant prévus pour transmettre un mouvement de l'élément de commande (39) aux freins (32, 33, 38).

2. Tube d'observation selon la revendication 1, **caractérisé en ce que** les freins (32, 33, 38) sont soumis à la pression de freinage au moyen d'un ou de plusieurs ressorts de compression (40).

3. Tube d'observation selon la revendication 2, **caractérisé en ce que** le ou les plusieurs ressorts de compression (40) agissent sur le système composé de coulisseaux (42) et de leviers coudés (43), l'élément de commande (39) agissant dans le système composé de coulisseaux (42) et de leviers coudés (43) entre le ou les plusieurs ressorts de compression (40) et le frein (38) immédiatement voisin du ou des plusieurs ressorts de compression (40).

4. Tube d'observation selon l'une des revendications 1 à 3, **caractérisé en ce qu'**à chacun des freins (32, 33, 38) est associée une articulation tournante (19, 20, 21) et ils peuvent être desserrés en appliquant une force parallèlement à un axe de rotation de l'articulation tournante sans renvoi de force supplémentaire.

5. Tube d'observation selon l'une des revendications 1 à 4, **caractérisé en ce que** le tube présente à chaque fois un tube intérieur (30, 34, 36) et un tube extérieur (31, 35, 37) au niveau des articulations tournantes (19, 20, 21) et **en ce que** les freins se composent de lamelles (32, 33, 38) avec des garnitures de friction et des éléments d'entraînement qui pénètrent en alternance dans le tube intérieur (30, 34, 36) et le tube extérieur (31, 35, 37).

6. Tube d'observation selon la revendication 5, **caractérisé en ce que** les freins (32, 33, 38) des différentes articulations tournantes (19, 20, 21) présentent des nombres de lamelles différents.

7. Tube d'observation selon l'une des revendications 1 à 6, **caractérisé en ce que** les freins présentent des rouleaux (53) qui s'étendent parallèlement à l'axe du tube (52) dans la zone de l'articulation tournante, lesquels sont poussés par des ressorts (54) contre le tube intérieur (52).

8. Tube d'observation selon l'une des revendications 1 à 7, **caractérisé en ce que** trois articulations tournantes (19, 20, 21) sont prévues, les axes de rotation de deux articulations tournantes étant espacés l'un de l'autre et parallèles et les axes de rotation d'une articulation tournante qui se trouve entre celles-ci est perpendiculaire aux deux autres axes de rotation.

9. Tube d'observation selon l'une des revendications 1 à 8, **caractérisé en ce que** des composants optiques (22, 23) destinés à transmettre un faisceau optique sont prévus dans l'espace intérieur du tube.

10. Tube d'observation selon l'une des revendications 1 à 9, **caractérisé en ce que** le système composé de coulisseaux et de leviers coudés est monté dans la paroi du tube.

11. Tube d'observation selon l'une des revendications 1 à 10, **caractérisé en ce que** le tube d'observation comprend un oculaire (26) qui est logé dans une partie tubulaire (18) sur laquelle est monté l'élément de commande (39).

12. Tube d'observation selon l'une des revendications 1 à 11, **caractérisé en ce qu'**il est prévu une paire de prismes (26) pour diviser un faisceau dans l'oculaire d'un tube d'oculaire monté ou à monter.
